# EUROPÄISCHE PATENTANMELDUNG

(11) **EP 4 270 038 A1**
(43) Veröffentlichungstag der Anmeldung: **01.11.2023**
(21) Anmeldenummer: 22170324.2
(22) Anmeldetag: 27.04.2022
(51) Int. Cl.: G01R 33/381, G01R 33/385, G01R 33/421, G01R 33/44

(54) **MAGNETANORDNUNG FÜR EINE MAGNETRESONANZVORRICHTUNG**

(71) Anmelder: Siemens Healthcare GmbH, 91052 Erlangen (DE)
(72) Erfinder: Dr. Krug, Andreas, 90762 Fürth (DE); Dr. Campagna, Swen, 91238 Engelthal (DE)

(57) **Zusammenfassung**

Die Erfindung betrifft eine Magnetanordnung (30) für eine Magnetresonanzvorrichtung (10) zum Erfassen von Magnetresonanzdaten eines Objekts, umfassend zumindest eine Gradienten-Anordnung (31) mit einer Mehrzahl von Gradientenspulen und einer Mehrzahl von Gradienten-Verstärkern, wobei die Mehrzahl von Gradienten-Verstärkern dazu ausgebildet ist, einen Stromfluss in der Mehrzahl von Gradientenspulen variabel einzustellen, wobei jede Gradientenspule (18) der zumindest einen Gradienten-Anordnung (31) jeweils elektrisch mit einem Gradienten-Verstärker (29) der zumindest einen Gradienten-Anordnung (31) verbunden ist und wobei die Magnetanordnung (30) dazu ausgebildet ist, mittels der Mehrzahl von Gradienten-Verstärkern abwechselnd ein im Wesentlichen homogenes Magnetfeld sowie ein magnetisches Gradientenfeld bereitzustellen. Ferner betrifft die Erfindung eine Magnetresonanzvorrichtung (10) zum Erfassen von Magnetresonanzdaten eines Objekts, umfassend eine Magnetanordnung (30) und eine Steuereinheit (22), welche dazu ausgebildet ist, eine Mehrzahl von Gradienten-Verstärkern anzusteuern einen Stromfluss in einer Mehrzahl von Gradientenspulen variabel einzustellen, um abwechselnd ein im Wesentlichen homogenes Magnetfeld sowie ein magnetisches Gradientenfeld in einer Bildgebungsregion der Magnetanordnung (30) bereitzustellen.

## Beschreibung

Die Erfindung betrifft eine Magnetanordnung für eine Magnetresonanzvorrichtung zum Erfassen von Magnetresonanzdaten eines Objekts. Ferner betrifft die Erfindung eine Magnetresonanzvorrichtung zum Erfassen von Magnetresonanzdaten eines Objekts, umfassend eine Magnetanordnung und eine Steuereinheit.

Die Magnetresonanztomografie ist ein bekanntes Bildgebungsverfahren, mit welchem Magnetresonanzbilder eines Inneren des Untersuchungsobjekts erzeugt werden können. Bei der Durchführung einer Magnetresonanzbildgebung wird das Untersuchungsobjekt üblicherweise in einem starken, statischen und homogenen Grundmagnetfeld (B0-Magnetfeld) einer Magnetresonanzvorrichtung positioniert. Das Grundmagnetfeld kann magnetische Feldstärken von 0,2 Tesla bis 7 Tesla aufweisen, sodass sich Kernspins des Untersuchungsobjekts entlang des Grundmagnetfeldes ausrichten. Um sogenannte Kernspinresonanzen auszulösen, werden hochfrequente Signale, sogenannte Anregungsimpulse (B1-Magnetfeld), in das Untersuchungsobjekt eingestrahlt. Jeder Anregungsimpuls bewirkt eine Abweichung einer Magnetisierung bestimmter Kernspins des Untersuchungsobjekts von dem Grundmagnetfeld um einen Betrag, welcher auch als Flipwinkel bekannt ist. Ein Anregungsimpuls kann dabei ein magnetisches Wechselfeld mit einer Frequenz aufweisen, welche der Larmorfrequenz bei der jeweiligen statischen Magnetfeldstärke entspricht. Die angeregten Kernspins können eine rotierende und abklingende Magnetisierung (Kernspinresonanz) aufweisen, welche sich mittels spezieller Antennen als Magnetresonanzsignal erfassen lässt. Zur räumlichen Kodierung der Kernspinresonanzen des Untersuchungsobjekts können dem Grundmagnetfeld magnetische Gradientenfelder überlagert werden.

Die empfangenen Magnetresonanzsignale werden typischerweise digitalisiert und als komplexe Werte (Magnetresonanzdaten) in einer k-Raum-Matrix gespeichert. Diese k-Raum-Matrix kann als Grundlage für eine Rekonstruktion von Magnetresonanzbildern sowie eine Bestimmung von Spektroskopiedaten verwendet werden. Die Rekonstruktion eines Magnetresonanzbilds erfolgt typischerweise mittels einer mehrdimensionalen FourierTransformation der k-Raum-Matrix.

Magnetanordnungen von Magnetresonanzvorrichtungen bestehen typischerweise aus Hauptmagneten zur Erzeugung eines statischen Magnetfeldes sowie Gradientenspulen zur Erzeugung von linear ansteigenden Magnetfeldern (magnetische Gradientenfelder). Während die Gradientenspulen häufig als resistive Spulen ausgestaltet sind, können die Hauptmagneten unterschiedliche Magnettypen, wie z. B. supraleitende Magnete, Permanentmagnete, Elektromagnete und dergleichen, aufweisen.

Es ist wünschenswert, kleinere bzw. dedizierte Magnetresonanzvorrichtungen mit niedrigen Magnetfeldstärken (< 1 Tesla) bereitzustellen, welche aufgrund geringerer Kosten und/oder kompakter Abmessungen insbesondere in kleineren Praxen und klinischen Einrichtungen, aber auch als dedizierte Systeme für Nicht-Radiologen (z.B. Zahnarztpraxen, Orthopädie, Augenkliniken, und dergleichen) eingesetzt werden können. Ein Betrieb von Magnetresonanzvorrichtungen, insbesondere solchen mit supraleitenden Hauptmagneten, erfordert jedoch eine aufwändige technische Infrastruktur, welche einen signifikanten Platzbedarf aufweist. Weiterhin bedingen magnetische Streufelder des Hauptmagneten eine erhebliche Nutzungseinschränkung im direkten Umfeld der Magnetresonanzvorrichtung. Bei konventionellen Magnetresonanzvorrichtungen sind Streufelder auch außerhalb des eigentlichen Scanbetriebs aktiv. Insbesondere bei supraleitenden Hauptmagneten muss das statische Magnetfeld dauerhaft aufrechterhalten werden, was eine kontinuierliche Kühlung bzw. einen dauerhaften Betrieb geeigneter Kühlgeräte erfordert.

Wird die Magnetresonanzvorrichtung jedoch nur eine gewisse Zeit pro Tag betrieben, so ist es wünschenswert, wenn der Hauptmagnet sowie erforderliche Kühlgeräte nur in dieser Zeit aktiv sind. Dadurch ließen sich Energiekosten für den Betrieb der Magnetresonanzvorrichtung einsparen.

Es ist daher eine Aufgabe der Erfindung, eine Magnetresonanzvorrichtung bereitzustellen, welche einen reduzierten Energiebedarf in Bereitschafts- bzw. Nichtnutzungsperioden aufweist.

Diese Aufgabe wird durch die Gegenstände der unabhängigen Patentansprüche erfindungsgemäß gelöst. Vorteilhafte Ausführungsformen und zweckmäßige Weiterbildungen sind Gegenstand der Unteransprüche.

Die erfindungsgemäße Magnetanordnung für eine Magnetresonanzvorrichtung zum Erfassen von Magnetresonanzdaten eines Objekts umfasst zumindest eine Gradienten-Anordnung mit einer Mehrzahl von Gradientenspulen und einer Mehrzahl von Gradienten-Verstärkern.

Die Magnetanordnung kann als eine Magnetfeld-erzeugende Einheit der Magnetresonanzvorrichtung verstanden werden. Die Magnetanordnung ist vorzugsweise dazu ausgebildet, ein Magnetfeld in einem Patientenaufnahmebereich der Magnetresonanzvorrichtung bereitzustellen. Es ist insbesondere vorstellbar, dass die Magnetanordnung dazu ausgebildet ist, Magnetfelder mit variierender Magnetfeldstärke und/oder Magnetfeldausrichtung in dem Patientenaufnahmebereich zu erzeugen. Die Magnetanordnung ist jedoch ebenso dazu ausgebildet, ein homogenes Magnetfeld in dem Patientenaufnahmebereich zu erzeugen.

Die Gradienten-Anordnung kann ein Teil eines Gradientensystems der Magnetanordnung darstellen. Das Gradientensystem kann dazu ausgebildet sein, magnetische Gradientenfelder in dem Patientenaufnahmebereich zu erzeugen. Insbesondere kann das Gradientensystem dazu ausgebildet sein, magnetische Gradientenfelder bereitzustellen, welche entlang unterschiedlicher Raumrichtungen ausgerichtet sind. Vorzugsweise ist das Gradientensystem dazu ausgebildet, zwei oder drei magnetische Gradientenfelder bereitzustellen, welche orthogonal zueinander ausgerichtet sind.

Die Magnetanordnung kann weiterhin ein Hochfrequenzsystem umfassen. Das Hochfrequenzsystem kann dazu ausgebildet sein, hochfrequente Signale in eine Bildgebungsregion auszusenden und/oder hochfrequente Signale (Magnetresonanzsignale) aus der Bildgebungsregion zu empfangen.

Das Gradientensystem kann eine oder mehrere Gradienten-Anordnungen umfassen. Eine Gradienten-Anordnung kann als eine Gruppe oder eine Anordnung von mehreren Gradientenspulen verstanden werden, welche dazu ausgebildet sind, durch ein Zusammenwirken abwechselnd ein homogenes Magnetfeld und ein magnetisches Gradientenfeld mit einer vorbestimmten Ausrichtung in der Bildgebungsregion bereitzustellen.

Die Mehrzahl von Gradienten-Verstärkern ist dazu ausgebildet, einen Stromfluss in der Mehrzahl von Gradientenspulen variabel einzustellen, wobei jede Gradientenspule der zumindest einen Gradienten-Anordnung jeweils elektrisch mit einem Gradienten-Verstärker der zumindest einen Gradienten-Anordnung verbunden ist.

Ein Gradienten-Verstärker ist dazu ausgebildet, einen Stromfluss in einer Gradientenspule zu erzeugen. Der Stromfluss in der Gradientenspule kann dabei mit einem vorbestimmten und/oder vorgegebenen elektrischen Signal, welches dem Gradienten-Verstärker bereitgestellt wird, korrespondieren. Vorzugsweise ist jeder Gradienten-Verstärker der zumindest einen Gradienten-Anordnung elektrisch mit einer Steuereinheit der Magnetresonanzvorrichtung verbunden. Es ist vorstellbar, dass ein oder mehrere Gradienten-Verstärker der zumindest einen Gradienten-Anordnung dazu ausgebildet sind, in Abhängigkeit eines Signals der Steuereinheit einen Stromfluss in einer oder mehrerer Gradientenspulen der zumindest einen Gradienten-Anordnung bereitzustellen. Die Stromflüsse in der einen oder den mehreren Gradientenspulen können dabei zeitgleich, aber auch unabhängig voneinander bereitgestellt werden.

In einer bevorzugten Ausführungsform weist die zumindest eine Gradienten-Anordnung eine erste Gradientenspule und eine zweite Gradientenspule auf. Die erste Gradientenspule kann dabei elektrisch mit einem ersten Gradienten-Verstärker verbunden sein, während die zweite Gradientenspule elektrisch mit einem zweiten Gradientenverstärker verbunden ist. Der erste Gradienten-Verstärker und der zweite Gradienten-Verstärker stellen vorzugsweise voneinander verschiedene Gradienten-Verstärker dar. Der erste Gradienten-Verstärker und der zweite Gradientenverstärker können elektrisch unverbunden sein. Vorzugsweise ist jede Gradientenspule der zumindest einen Gradienten-Anordnung elektrisch mit genau einem Gradienten-Verstärker der zumindest einen Gradienten-Anordnung verbunden.

Es ist jedoch vorstellbar, dass die zumindest eine Gradienten-Anordnung eine Mehrzahl von Gradientenspulen, beispielsweise vier, sechs, acht, zehn oder mehr, Gradientenspulen aufweist. In diesem Fall kann ein Gradientenverstärker auch elektrisch mit einer Mehrzahl von Gradientenspulen verbunden sein, um einen Stromfluss in der Mehrzahl von Gradientenspulen zu erzeugen.

Die erfindungsgemäße Magnetanordnung ist dazu ausgebildet, mittels der Mehrzahl von Gradienten-Verstärkern abwechselnd ein im Wesentlichen homogenes Magnetfeld sowie ein magnetisches Gradientenfeld bereitzustellen. Beispielsweise kann die Mehrzahl von Gradienten-Verstärkern der zumindest einen Gradienten-Anordnung dazu ausgebildet sein, einen Stromfluss in der Mehrzahl von Gradientenspulen anzupassen, um anstelle des homogenen Magnetfelds das magnetische Gradientenfeld zu erzeugen.

Es ist jedoch ebenso vorstellbar, dass die erfindungsgemäße Magnetanordnung zumindest eine weitere Gradienten-Anordnung, z. B. eine zweite Gradienten-Anordnung und/oder eine dritte Gradienten-Anordnung, umfasst. In diesem Fall kann eine erste Gradienten-Anordnung dazu ausgebildet sein, das im Wesentlichen homogene Magnetfeld bereitzustellen, während eine weitere Gradienten-Anordnung dazu ausgebildet ist, das magnetische Gradientenfeld bereitzustellen. Das Bereitstellen des magnetischen Gradientenfelds kann somit auch ein Überlagern des im Wesentlichen homogenen Magnetfelds mit dem magnetischen Gradientenfeld umfassen.

Ein magnetisches Gradientenfeld kann durch einen linearen oder nichtlinearen Magnetfeldstärkeverlauf in zumindest einer Raumrichtung gekennzeichnet sein. Das magnetische Gradientenfeld kann weiterhin durch eine Überlagerung des im Wesentlichen homogenen Magnetfelds mit dem magnetischen Gradientenfeld charakterisiert sein. Es ist weiterhin vorstellbar, dass das magnetische Gradientenfeld das im Wesentlichen homogene Magnetfeld ersetzt.

Die erfindungsgemäße Magnetanordnung ist vorzugsweise dazu ausgebildet, das im Wesentlichen homogene Magnetfeld und/oder das magnetische Gradientenfeld nach Bedarf bereitzustellen. Das im Wesentlichen homogene Magnetfeld und das magnetische Gradientenfeld können beispielsweise in Abhängigkeit einer Magnetresonanzuntersuchung, einer Bildgebungssequenz, eines Bildgebungsprotokolls und/oder eines Bildgebungsparameters abwechselnd bereitgestellt werden.

Eine Magnetfeldstärke des im Wesentlichen homogenen Magnetfelds beträgt vorzugsweise einige hundert Millitesla. Beispielsweise liegt die Magnetfeldstärke des im Wesentlichen homogenen Magnetfelds zwischen 0,1 T und 0,5 T oder zwischen 0,1 T und 0,3 T. Eine Gradientenstärke des magnetischen Gradientenfelds liegt beispielsweise in einem Bereich von einigen 10 mT/m, insbesondere zwischen 10 mT/m und 60 mT/m oder vorzugsweise zwischen 10 mT/m und 30 mT/m.

Vorzugsweise weist die Magnetanordnung und/oder eine Magnetresonanzvorrichtung, welche die Magnetanordnung umfasst, eine Steuereinheit auf. Die Steuereinheit kann dazu ausgebildet sein, die zumindest eine Gradienten-Anordnung und/oder weitere Gradienten-Anordnungen anzusteuern, das im Wesentlichen homogene Magnetfeld oder das magnetische Gradientenfeld bereitzustellen.

Die erfindungsgemäße Magnetanordnung kann insbesondere einen konventionellen Hauptmagneten zur Erzeugung eines statischen, homogenen Magnetfelds (B0) ersetzen. Dies kann bedeuten, dass die erfindungsgemäße Magnetanordnung ohne einen konventionellen oder separaten Hauptmagneten imstande ist, ein homogenes Magnetfeld bereitzustellen. Das durch die erfindungsgemäße Magnetanordnung bereitgestellte homogene Magnetfeld kann vorteilhaft vorübergehend oder für längere Zeiträume außer Betrieb genommen werden, ohne dass ein aufwändiger Ramp-down oder Ramp-Up, wie von supraleitenden Magneten bekannt, erforderlich ist.

Mittels der erfindungsgemäßen Magnetanordnung lassen sich Betriebszeiten der Magnetresonanzvorrichtung auf vorteilhafte Weise an einen tatsächlichen Bedarf von Magnetresonanzuntersuchungen anpassen. Insbesondere lassen sich Standby- oder Bereitschaftsperioden, in denen konventionelle Magnetresonanzvorrichtungen einen relativen hohen Energiebedarf aufweisen, vermeiden.

Weiterhin lassen sich Aufwände für die Fertigung und/oder die Installation einer Magnetresonanzvorrichtung durch Verwendung der erfindungsgemäßen Magnetanordnung vorteilhaft vermeiden, da auf konventionelle Hauptmagneten mit entsprechenden Kühlgeräten verzichtet werden kann.

Durch den Verzicht auf einen konventionellen Hauptmagneten lassen sich auch technische Einrichtungen zur Schirmung des Hauptmagneten vermeiden, wodurch Kosten und Abmessungen der Magnetresonanzvorrichtung vorteilhaft gesenkt werden können. Ferner kann eine Nutzung eines Untersuchungsraumes, in welchem die Magnetresonanzvorrichtung untergebracht ist, durch Vermeidung eines konventionellen Hauptmagneten mit einem andauernd aktiven Magnetfeld auf vorteilhafte Weise verbessert und/oder vereinfacht werden.

In einer bevorzugten Ausführungsform der erfindungsgemäßen Magnetanordnung umfasst die zumindest eine Gradienten-Anordnung eine erste Gradientenspule und eine zweite Gradientenspule.

Vorzugsweise weist die zumindest eine Gradienten-Anordnung genau zwei Gradientenspulen auf. Die zumindest eine Gradienten-Anordnung kann aber auch mehr als zwei Gradientenspulen, insbesondere drei oder vier Gradientenspulen, umfassen.

Die erste Gradientenspule und die zweite Gradientenspule sind im Wesentlichen kreisförmig und planar in parallel ausgerichteten, voneinander getrennten Ebenen angeordnet, sodass eine Projektion einer von der ersten Gradientenspule umschlossenen, ersten Fläche entlang eines Normalenvektors der ersten Fläche und eine von der zweiten Gradientenspule umschlossenen, zweiten Fläche, eine nichtleere Schnittmenge aufweisen.

Die Gradientenspulen der zumindest einen Gradienten-Anordnung können insbesondere als Teile einer Helmholtz- oder Maxwell-Anordnung ausgestaltet sein. Im Falle einer Maxwell-Anordnung kann die zumindest eine Gradienten-Anordnung insbesondere eine dritte Gradientenspule aufweisen, welche bevorzugt zwischen der ersten Gradientenspule und der zweiten Gradientenspule angeordnet ist.

Vorzugsweise weist die Magnetanordnung eine hohlzylinderförmige Gestalt auf. Die erste Gradientenspule und die zweite Gradientenspule können dabei derart angeordnet sein, dass Mittelpunkte der von der ersten Gradientenspule und der zweiten Gradientenspule umschlossenen Fläche auf einer Zylinderachse der hohlzylinderförmigen Magnetanordnung angeordnet sind. Die erste Gradientenspule und die zweite Gradientenspule sind vorzugsweise an gegenüberliegenden Enden der hohlzylinderförmigen Magnetanordnung positioniert. Es ist vorstellbar, dass die erste Gradientenspule und die zweite Gradientenspule einen Patientenaufnahmebereich, welcher von der zylinderförmigen Magnetanordnung gebildet wird, entlang einer Umfangsrichtung umschließen.

Die Gestalt der Magnetanordnung kann jedoch auch von einem Hohlzylinder abweichen. In diesem Fall sind die erste Gradientenspule und die zweite Gradientenspule vorzugsweise auf zwei gegenüberliegenden Seiten des Patientenaufnahmebereichs angeordnet. Die Magnetresonanzvorrichtung kann hierbei insbesondere als ein "C"-Scanner bzw. ein "offener" Scanner ausgestaltet sein.

Die erste Gradientenspule ist elektrisch mit einem ersten Gradienten-Verstärker verbunden und die zweite Gradientenspule ist elektrisch mit einem zweiten Gradienten-Verstärker verbunden. Die erste Gradientenspule ist vorzugsweise elektrisch von der zweiten Gradientenspule getrennt.

Der erste Gradienten-Verstärker kann dazu ausgebildet sein, einen Stromfluss in der ersten Gradientenspule einzustellen. Vorzugsweise ist der erste Gradienten-Verstärker dazu ausgebildet, eine Stromstärke und/oder eine Spannung, z. B. in Abhängigkeit eines Steuersignals einer Steuereinheit, einer Recheneinheit oder dergleichen, einzustellen und/oder zu verändern. Der zweite Gradienten-Verstärker kann gleichermaßen dazu ausgebildet sein, eine Stromstärke und/oder eine Spannung eines Stromflusses durch die zweite Gradientenspule einzustellen. Die Stromflüsse durch die erste Gradientenspule und die zweite Gradientenspule können ein gleiches Vorzeichen oder unterschiedliche Vorzeichen aufweisen. Die erste Gradientenspule und die zweite Gradientenspule sind vorzugsweise dazu ausgebildet, mittels des ersten Gradienten-Verstärkers und des zweiten Gradienten-Verstärkers abwechselnd ein homogenes Magnetfeld (B0-Feld) und ein magnetisches Gradientenfeld bereitzustellen. Das magnetische Gradientenfeld kann hierbei insbesondere entlang der Zylinderachse (z. B. einer Z-Richtung) der hohlzylinderförmigen Magnetanordnung ausgerichtet sein.

Durch das Bereitstellen der ersten Gradientenspule mit dem ersten Gradienten-Verstärker und der zweiten Gradientenspule mit dem zweiten Gradienten-Verstärker lässt sich ein Stromfluss durch die erste Gradientenspule auf vorteilhafte Weise unabhängig von dem Stromfluss durch die zweite Gradientenspule einstellen. Dadurch kann ein homogenes Magnetfeld oder ein magnetisches Gradientenfeld in Abhängigkeit von Erfordernissen einer Bildgebungssequenz in einer Bildgebungsregion eingestellt werden. Die Bildgebungsregion kann im Wesentlichen mit einem Patientenaufnahmebereich einer Magnetresonanzvorrichtung für menschliche oder tierische Patienten übereinstimmen. Die Magnetresonanzvorrichtung kann aber ebenso für die Untersuchung von beliebigen Objekten, wie z. B. archäologischen Fundstücken oder Lebensmitteln, angepasst sein und eine entsprechend dimensionierte Bildgebungsregion aufweisen.

In einer weiteren Ausführungsform umfasst die erfindungsgemäße Magnetanordnung ferner eine zweite Gradienten-Anordnung mit einer Mehrzahl von zweiten Gradientenspulen und einer Mehrzahl von zweiten Gradienten-Verstärkern, wobei wenigstens zwei Gradientenspulen der Mehrzahl von zweiten Gradientenspulen jeweils elektrisch mit einem Gradienten-Verstärker der Mehrzahl von zweiten Gradienten-Verstärkern verbunden sind.

Vorzugsweise ist ein erster Gradienten-Verstärker der zweiten Gradienten-Anordnung elektrisch mit einer ersten Gradientenspule der zweiten Gradienten-Anordnung verbunden, während ein zweiter Gradienten-Verstärker der zweiten Gradienten-Anordnung elektrisch mit einer zweiten Gradientenspule der zweiten Gradienten-Anordnung verbunden ist. Die erste Gradientenspule und die zweite Gradientenspule der zweiten Gradienten-Anordnung sind vorzugsweise elektrisch voneinander getrennt.

Die Magnetanordnung ist dazu ausgebildet, mittels der Mehrzahl von zweiten Gradienten-Verstärkern abwechselnd ein im Wesentlichen homogenes Magnetfeld sowie ein zweites magnetisches Gradientenfeld bereitzustellen, wobei sich eine Ausrichtung des zweiten magnetischen Gradientenfelds von einer Ausrichtung des magnetischen Gradientenfelds unterscheidet.

In einer bevorzugten Ausführungsform ist das zweite magnetische Gradientenfeld im Wesentlichen orthogonal zu dem magnetischen Gradientenfeld ausgerichtet.

Wie oben beschrieben, können der erste Gradienten-Verstärker und der zweite Gradientenverstärker der zweiten Gradienten-Anordnung dazu ausgebildet sein, in der ersten Gradientenspule und der zweiten Gradientenspule der zweiten Gradienten-Anordnung symmetrischen Stromflüsse mit gleichem Vorzeichen einzustellen. Das dadurch von der zweiten Gradienten-Anordnung erzeugte Magnetfeld kann insbesondere ein homogenes Magnetfeld sein. Weiterhin sind der erste Gradienten-Verstärker und der zweite Gradientenverstärker der zweiten Gradienten-Anordnung dazu ausgebildet, in der ersten Gradientenspule und der zweiten Gradientenspule der zweiten Gradienten-Anordnung unterschiedliche Stromflüsse und/oder Stromflüsse mit unterschiedlichem Vorzeichen einzustellen. Das dadurch erzeugte Magnetfeld kann insbesondere ein zweites magnetisches Gradientenfeld darstellen, welches im Wesentlichen orthogonal zu dem magnetischen Gradientenfeld ausgerichtet ist.

In einer weiteren Ausführungsform umfasst die erfindungsgemäße Magnetanordnung ferner eine dritte Gradienten-Anordnung mit einer Mehrzahl von dritten Gradientenspulen und einer Mehrzahl von dritten Gradienten-Verstärkern. Wenigstens zwei Gradientenspulen der Mehrzahl von dritten Gradientenspulen sind jeweils elektrisch mit einem Gradienten-Verstärker der Mehrzahl von dritten Gradienten-Verstärkern verbunden. Entsprechend einer oben beschriebenen Ausführungsform kann ein erster Gradienten-Verstärker der dritten Gradienten-Anordnung elektrisch mit einer ersten Gradientenspule der dritten Gradienten-Anordnung verbunden sein, während ein zweiter Gradienten-Verstärker der dritten Gradienten-Anordnung elektrisch mit einer zweiten Gradientenspule der dritten Gradienten-Anordnung verbunden ist. Die erste Gradientenspule und die zweite Gradientenspule der dritten Gradienten-Anordnung können insbesondere elektrisch voneinander getrennt sein.

Die Magnetanordnung ist dazu ausgebildet, mittels der Mehrzahl von dritten Gradienten-Verstärkern abwechselnd ein im Wesentlichen homogenes Magnetfeld sowie ein drittes magnetisches Gradientenfeld bereitzustellen, wobei sich eine Ausrichtung des dritten magnetischen Gradientenfelds von einer Ausrichtung des magnetischen Gradientenfelds und/oder des zweiten magnetischen Gradientenfelds unterscheidet.

In einer bevorzugten Ausführungsform ist das dritte magnetische Gradientenfeld im Wesentlichen orthogonal zu dem magnetischen Gradientenfeld und/oder dem zweiten magnetischen Gradientenfeld ausgerichtet.

Der erste Gradienten-Verstärker und der zweite Gradientenverstärker der dritten Gradienten-Anordnung können dazu ausgebildet sein, symmetrische Stromflüsse mit gleichen Vorzeichen in der ersten Gradientenspule und der zweiten Gradientenspule der dritten Gradienten-Anordnung einzustellen. Das dadurch von der dritten Gradienten-Anordnung erzeugte Magnetfeld kann insbesondere ein homogenes Magnetfeld sein. Es ist ebenso vorstellbar, dass der erste Gradienten-Verstärker und der zweite Gradientenverstärker der dritten Gradienten-Anordnung dazu ausgebildet sind, in der ersten Gradientenspule und der zweiten Gradientenspule der dritten Gradienten-Anordnung unsymmetrische Stromflüsse, wie z. B. unterschiedliche Stromflüsse und/oder Stromflüsse mit unterschiedlichem Vorzeichen, einzustellen. Das dadurch erzeugte Magnetfeld kann insbesondere ein drittes magnetisches Gradientenfeld sein, welches orthogonal zu dem magnetischen Gradientenfeld und dem zweiten magnetischen Gradientenfeld ausgerichtet ist.

Durch das Bereitstellen der zweiten Gradienten-Anordnung und/oder der dritten Gradienten-Anordnung lässt sich eine magnetische Feldstärke eines von der zumindest einen Gradienten-Anordnung bereitgestellten, homogenen Magnetfelds auf vorteilhafte Weise durch Überlagerung mit einem von der zweiten Gradienten-Anordnung und/oder der dritten Gradienten-Anordnung bereitgestellten, homogenen Magnetfeld erhöhen. Dadurch können technische Anforderungen der zumindest einen Gradienten-Anordnung auf vorteilhafte Weise reduziert werden. Weiterhin können mittels der zweiten Gradienten-Anordnung und/oder der dritten Gradienten-Anordnung zumindest kurzzeitig ein zweites magnetisches Gradientenfeld und/oder ein drittes magnetisches Gradientenfeld bereitgestellt werden, welche sich vorteilhaft für eine räumliche Zuordnung empfangener Magnetresonanzsignale verwenden lassen.

In einer bevorzugten Ausführungsform der erfindungsgemäßen Magnetanordnung ist die Mehrzahl von zweiten Gradientenspulen und/oder die Mehrzahl von dritten Gradientenspulen als Sattelspulen ausgestaltet.

Sattelspulen können die Form einer Zylinderschale, insbesondere einer Halbzylinderschale, aufweisen. Sattelspulen können elektrisch leitfähige Signalleiter umfassen, welche in einem "Fingerabdruck" Muster oder einem vergleichbaren Wicklungsmuster (fingerprint pattern) angeordnet sein können. Die Sattelspulen können auch als sogenannte Golay-Spulen ausgestaltet sein. Vorzugsweise ist die zweite Mehrzahl von Gradientenspulen und die dritte Mehrzahl von Gradientenspulen derart in der Magnetanordnung angeordnet, dass die Sattelspulen ein zylinderförmiges Volumen einschließen, welches im Wesentlichen mit der Bildgebungsregion der Magnetanordnung übereinstimmt.

In einer alternativen Ausführungsform der erfindungsgemäßen Magnetanordnung ist die Mehrzahl von zweiten Gradientenspulen und/oder die Mehrzahl von dritten Gradientenspulen als Segmentspulen ausgestaltet. Eine Segmentspule kann derart ausgestaltet sein, dass ein Strom eines felderzeugenden Leiters der Segmentspule auf einer Zylinderoberfläche mit größerem Radius zurückfließt. Dies kann bedeuten, dass die Segmentspule Rückleiter (Sekundärleiter) aufweist, welche im Wesentlichen parallel versetzt zu Nutzleitern (Primärleitern) in einer von der Bildgebungsregion abgewandten Seite der Nutzleiter angeordnet sind.

Durch das Bereitstellen einer Segmentspule können die Rückleiter auf der äußeren Zylinderoberfläche auf vorteilhafte Weise axial verschoben angeordnet sein, um die Homogenität eines erzeugten Magnetfelds zu verbessern.

Gemäß einer weiteren Ausführungsform weist die erfindungsgemäße Magnetanordnung eine im Wesentlichen hohlzylinderförmige Gestalt auf.

Die Magnetanordnung kann insbesondere die Gestalt eines Tubus bzw. eines Hohlzylinders aufweisen. Die hohlzylinderförmige Magnetanordnung ist vorzugsweise derart ausgestaltet, dass sie ein Bildgebungsvolumen innerhalb der Bildgebungsregion bzw. des Patientenaufnahmebereichs entlang einer Umfangsrichtung umschließt.

Zumindest eine Gradientenspule der zweiten Gradienten-Anordnung umfasst einen ersten Spulenabschnitt und einen zweiten Spulenabschnitt. Der erste Spulenabschnitt und der zweite Spulenabschnitt sind disjunkt voneinander entlang einer axialen Richtung der hohlzylinderförmigen Magnetanordnung aufeinanderfolgend angeordnet.

Der erste Spulenabschnitt kann beispielsweise einen separaten Wicklungsabschnitt oder einen separaten Teilabschnitt der zumindest einen Gradientenspule darstellen. Dies kann bedeuten, dass der erste Spulenabschnitt und der zweite Spulenabschnitt voneinander beabstandet sind. Eine Wicklung des ersten Spulenabschnitts ist vorzugsweise räumlich von einer Wicklung des zweiten Spulenabschnitts der zumindest einen Gradientenspule getrennt. Die zumindest eine Gradientenspule kann weiterhin aus einer Mehrzahl von disjunkten Spulenabschnitten, wie z. B. zwei, drei, vier oder mehr Spulenabschnitten, bestehen.

Vorzugsweise sind der erste Spulenabschnitt und der zweite Spulenabschnitt auf einer gemeinsamen Zylinderhälfte der hohlzylinderförmigen Magnetanordnung angeordnet. Es ist insbesondere vorstellbar, dass der erste Spulenabschnitt und der zweite Spulenabschnitt auf zwei im Wesentlichen spiegelsymmetrischen Zylinderabschnitten der hohlzylinderförmigen Magnetanordnung angeordnet sind oder die hohlzylinderförmige Magnetanordnung in zwei im Wesentlichen spiegelsymmetrische Zylinderabschnitte unterteilt.

Der erste Spulenabschnitt und der zweite Spulenabschnitt sind gemeinsam elektrisch mit genau einem Gradienten-Verstärker der zweiten Gradienten-Anordnung verbunden.

In einer Ausführungsform kann auch eine Gradientenspule der dritten Gradienten-Anordnung einen ersten Spulenabschnitt und einen zweiten Spulenabschnitt umfassen, wobei der erste Spulenabschnitt und der zweite Spulenabschnitt disjunkt voneinander entlang einer axialen Richtung der hohlzylinderförmigen Magnetanordnung aufeinanderfolgend angeordnet sind. Vorzugsweise sind der erste Spulenabschnitt und der zweite Spulenabschnitt der Gradientenspule der dritten Gradienten-Anordnung elektrisch mit genau einem Gradienten-Verstärker der dritten Gradienten-Anordnung verbunden. Der erste Spulenabschnitt und der zweite Spulenabschnitt der Gradientenspule der dritten Gradienten-Anordnung können analog zu den Spulenabschnitten der zweiten Gradienten-Anordnung angeordnet sein. Vorzugsweise sind die Spulenabschnitte der dritten Gradienten-Anordnung um einen Winkel von etwa 90° versetzt gegenüber den Spulenabschnitten der zweiten Gradienten-Anordnung angeordnet.

Ferner kann auch eine zweite Gradientenspule der zweiten Gradienten-Anordnung einen ersten Spulenabschnitt und einen zweiten Spulenabschnitt gemäß einer oben beschriebenen Ausführungsform aufweisen. Es ist vorstellbar, dass der erste Spulenabschnitt der zumindest einen Gradientenspule und der erste Spulenabschnitt der weiteren Gradientenspule entlang der axialen Richtung der hohlzylinderförmigen Magnetanordnung gegenüberliegend angeordnet sind. Gleichermaßen können der zweite Spulenabschnitt der zumindest einen Gradientenspule und der zweite Spulenabschnitt der weiteren Gradientenspule entlang der axialen Richtung der hohlzylinderförmigen Magnetanordnung gegenüberliegend angeordnet sein.

In einer alternativen Ausführungsform weist die Magnetanordnung eine von einem Hohlzylinder abweichende Gestalt auf. Beispielsweise kann die Magnetanordnung auch C-förmig, U-förmig oder V-förmig ausgestaltet sein. Es ist weiterhin vorstellbar, dass die von der Magnetanordnung gebildete Patientenaufnahmeregion an eine Form einer Körperregion eines Patienten angepasst ist. Beispielsweise kann die Magnetanordnung einem Kopf, einer Hüfte, einer Schulter, einer Brust, einem Knie, einem Arm und/oder einem Bein eines Patienten nachgeformt sein.

Der erste Spulenabschnitt und/oder der zweite Spulenabschnitt der zweiten Gradienten-Anordnung und/oder der dritten Gradienten-Anordnung können weiterhin in eine Anzahl von zwei, drei, vier oder mehr Teilabschnitten unterteilt sein. Vorzugsweise ist die Mehrzahl von Teilabschnitten des ersten Spulenabschnitts entlang der axialen Richtung der hohlzylinderförmigen Magnetanordnung aufeinanderfolgend angeordnet. Gleichermaßen kann auch die Mehrzahl von Teilabschnitten des zweiten Spulenabschnitts entlang der axialen Richtung der hohlzylinderförmigen Magnetanordnung aufeinanderfolgend angeordnet sein.

Die Mehrzahl von Teilabschnitten des ersten Spulenabschnitts ist elektrisch mit einem ersten Gradienten-Verstärker verbunden.

Gleichermaßen kann die Mehrzahl von Teilabschnitten des zweiten Spulenabschnitts elektrisch mit einem zweiten Gradienten-Verstärker verbunden sein. Der erste Gradientenverstärker und der zweite Gradienten-Verstärker unterscheiden sich dabei voneinander.

Durch das Bereitstellen von Spulenabschnitten mit einer Mehrzahl von Teilabschnitten lässt sich ein Fertigungsaufwand der Magnetanordnung auf vorteilhafte Weise reduzieren. Weiterhin lässt sich eine Gradienten-Anordnung mit einer Mehrzahl von Spulenabschnitten, aber auch ein mittels der Gradienten-Anordnung erzeugtes Magnetfeld, verbessert an eine Geometrie dedizierter Magnetresonanzvorrichtung für eine Magnetresonanzuntersuchung spezifischer Objekte anpassen.

In einer weiteren Ausführungsform der erfindungsgemäßen Magnetanordnung weist zumindest eine Gradientenspule der zumindest einen Gradienten-Anordnung, der zweiten Gradienten-Anordnung und/oder der dritten Gradienten-Anordnung eine Sekundärspule auf. Die Sekundärspule ist im Wesentlichen parallel zu der zumindest einen Gradientenspulen angeordnet.

Eine dreidimensionale Gestalt, eine Abmessung, eine geometrische Form, aber auch ein Wicklungsmuster der Sekundärspule können im Wesentlichen mit einer dreidimensionalen Gestalt, einer Abmessung, einer geometrischen Form und/oder einem Wicklungsmuster der zumindest einen Gradientenspule übereinstimmen.

Die Sekundärspule begrenzt die zumindest eine Gradientenspule in einer von der Bildgebungsregion der Magnetanordnung abgewandten Richtung. Dies kann bedeuten, dass die Sekundärspule die zumindest eine Gradientenspule auf einer von dem Bildgebungsvolumen abgewandten Seite umschließt oder umgreift. Vorzugsweise sind die Sekundärspule und die zumindest eine Gradientenspule dabei voneinander beabstandet. Ein Abstand zwischen der Sekundärspule und der zumindest einen Gradientenspule kann beispielsweise mehrere Millimeter oder mehrere Zentimeter betragen.

Besteht die zumindest eine Gradientenspule aus mehreren Spulenabschnitten und/oder einer Mehrzahl von Teilabschnitten , so kann auch die Sekundärspule eine entsprechende Anzahl von Spulenabschnitten und/oder Teilabschnitten aufweisen.

Vorzugsweise sind die Sekundärspule und die zumindest eine Gradientenspule gemeinsam elektrisch mit einem Gradienten-Verstärker verbunden. Dies kann bedeuten, dass ein Stromfluss durch die Sekundärspule und die zumindest eine Gradientenspule gemeinsam mittels eines Ausgangssignals des Gradienten-Verstärkers einstellbar ist.

Durch das Bereitstellen einer Sekundärspule lässt sich eine magnetische Feldstärke eines erzeugten homogenen Magnetfelds oder eines magnetischen Gradientenfelds der erfindungsgemäßen Magnetanordnung auf vorteilhafte Weise erhöhen. Beispielsweise kann eine Sekundärspule der erfindungsgemäßen Magnetanordnung im Vergleich zu konventionellen Gradientenspulen, bei denen die Sekundärspulen ein erzeugtes magnetisches Gradientenfeld außerhalb der Gradientenspule (typischerweise in Richtung des Hauptmagneten) schirmen, auf vorteilhafte Weise dazu dienen, eine magnetische Feldstärke in der Bildgebungsregion zu verstärken. Auf eine elektromagnetische Schirmung der Gradienten-Anordnung(en) kann dabei vorteilhaft verzichtet werden.

In einer bevorzugten Ausführungsform der erfindungsgemäßen Magnetanordnung ist die zumindest eine Gradienten-Anordnung, die zweite Gradienten-Anordnung und/oder die dritte Gradienten-Anordnung elektromagnetisch ungeschirmt.

In konventionellen Magnetresonanzvorrichtungen schützt eine elektromagnetische Schirmung unter anderem den Hauptmagneten, aber auch eine Umgebung der Magnetresonanzvorrichtung, vor elektromagnetischen Feldern. Insbesondere ist bei konventionellen Magnetresonanzvorrichtungen eine Schirmung zwischen dem Gradientensystem und dem (supraleitenden) Hauptmagneten angeordnet. Dadurch können Auswirkungen der Gradientenspulen auf den Hauptmagneten, wie z. B. Wirbelströme (Eddy Ströme), zu reduzieren oder zu vermeiden.

Die elektromagnetische Schirmung konventioneller Magnetresonanzvorrichtungen weist üblicherweise elektrisch leitfähige Strukturen, wie z. B. hochleitenden Metalle oder supraleitende Materialien, auf. Auf einen Einsatz solcher elektrisch leitfähigen Strukturen wird bei der erfindungsgemäßen Magnetanordnung bevorzugt verzichtet, da kein Hauptmagnet vorliegt. Ferner kann die erfindungsgemäße Magnetanordnung in einem Bereitschafts- oder Standby-Betrieb vorteilhafte in einen Magnetfeld-freien Zustand überführt werden. Somit wird eine Nutzung des Untersuchungsraums der Magnetresonanzvorrichtung vorteilhaft auch ohne Verwendung einer elektromagnetischen Schirmung ermöglicht.

Gemäß einer bevorzugten Ausführungsform ersetzen die zumindest eine Gradienten-Anordnung, die zweite Gradienten-Anordnung und/oder die dritte Gradienten-Anordnung der erfindungsgemäßen Magnetanordnung einen Hauptmagneten zur Erzeugung eines statischen, homogenen Magnetfelds.

Ein Hauptmagnet konventioneller Magnetresonanzvorrichtungen ist insbesondere durch die Erzeugung eines statischen Magnetfeldfelds gekennzeichnet. Das statische Magnetfeld liegt typischerweise kontinuierlich vor, z. B. auch wenn sich die Magnetresonanzvorrichtung in einem Standby-Modus befindet. Ein ordnungsgemäßes Herunterfahren des Hauptmagneten ist dabei mit einem hohen Aufwand verbunden. Gleichermaßen ist eine Wiederinbetriebnahme des Hauptmagneten mit einem hohen Zeitaufwand verbunden, da sich der Hauptmagnet bei einer Außerbetriebnahme erwärmt und zunächst wieder auf ein supraleitendes Temperaturniveau heruntergekühlt werden muss.

Hingegen ist das mittels der zumindest einen Gradienten-Anordnung, der zweiten Gradienten-Anordnung und/oder der dritten Gradienten-Anordnung der erfindungsgemäßen Magnetanordnung erzeugte Magnetfeld in bereits kurzer Zeit außer Betrieb genommen bzw. abgeschaltet.

Ein Ersetzen des Hauptmagneten durch die zumindest eine Gradienten-Anordnung, die zweite Gradienten-Anordnung und/oder die dritte Gradienten-Anordnung kann insbesondere bedeuten, dass auf den Hauptmagneten verzichtet wird. Die erfindungsgemäße Magnetanordnung, aber auch die erfindungsgemäße Magnetresonanzvorrichtung, können insbesondere dadurch charakterisiert sein, dass sie keinen Hauptmagneten aufweisen oder auf einen Hauptmagneten verzichten. Vorzugsweise erfolgt das Bereitstellen eines statischen, homogenen Magnetfelds bei der erfindungsgemäßen Magnetanordnung bzw. der erfindungsgemäßen Magnetresonanzvorrichtung mittels der zumindest einen Gradienten-Anordnung, der zweiten Gradienten-Anordnung und/oder der dritten Gradienten-Anordnung.

Durch den Verzicht auf die elektromagnetische Schirmung, aber auch den Hauptmagneten, können ein Gewicht, ein Fertigungsaufwand, aber auch ein Materialeinsatz, der erfindungsgemäßen Magnetanordnung auf vorteilhafte Weise gegenüber einer konventionellen Magnetanordnung reduziert werden.

Die erfindungsgemäße Magnetresonanzvorrichtung zum Erfassen von Magnetresonanzdaten eines Objekts umfasst eine Magnetanordnung gemäß einer oben beschriebenen Ausführungsform und eine Steuereinheit, welche dazu ausgebildet ist, eine Mehrzahl von Gradienten-Verstärkern anzusteuern einen Stromfluss in einer Mehrzahl von Gradientenspulen variabel einzustellen, um abwechselnd ein im Wesentlichen homogenes Magnetfeld sowie ein magnetisches Gradientenfeld in einer Bildgebungsregion der Magnetanordnung bereitzustellen.

Ein Objekt kann beispielsweise ein menschlicher oder tierischer Körper, aber auch ein beliebiger Gegenstand sein. Vorzugsweise ist das Objekt ein menschlicher Patient.

Die Steuereinheit der Magnetresonanzvorrichtung weist eine Signalverbindung mit der Mehrzahl von Gradienten-Verstärkern auf. Vorzugsweise ist die Steuereinheit dazu ausgebildet, einzelne Gradienten-Verstärker der Mehrzahl von Gradienten-Verstärkern individuell mittels der Signalverbindung anzusteuern, um den Stromfluss in einer elektrisch mit den Gradienten-Verstärkern verbundenen Gradientenspulen einzustellen oder vorzugeben.

Die Steuereinheit kann in die Magnetresonanzvorrichtung integriert sein oder als eigenständige Komponente ausgeführt sein. Vorzugsweise weist die Steuereinheit eine Signalverbindung mit einer Recheneinheit der Magnetresonanzvorrichtung auf. Es ist vorstellbar, dass die Steuereinheit eigenständig oder in Abhängigkeit eines Signals der Recheneinheit dazu ausgebildet ist, den Stromfluss durch die zumindest eine Gradienten-Anordnung an eine gewünschte Bildgebungssequenz oder ein Magnetresonanzdaten-Erfassungsprotokoll anzupassen. Die gewünschte Bildgebungssequenz oder das Magnetresonanzdaten-Erfassungsprotokoll können hierbei durch einen Nutzer der Magnetresonanzvorrichtung vorgegeben sein, beispielsweise um eine Magnetresonanzuntersuchung des Objekts durchzuführen.

Die erfindungsgemäßen Magnetresonanzuntersuchung teilt die Vorteile der erfindungsgemäßen Magnetanordnung. Durch das Bereitstellen der Steuereinheit lassen sich die Stromflüsse durch die Gradienten-Anordnung der Magnetanordnung auf vorteilhafte Weise an einen Bedarf einer Magnetresonanzuntersuchung anapassen. Beispielsweise lässt sich eine magnetische Feldstärke eines erzeugten homogenen Magnetfelds in Abhängigkeit des Objekts und/oder der durchzuführenden Magnetresonanzuntersuchung anpassen. Dadurch können ein Energiebedarf der Magnetresonanzvorrichtung, aber auch eine Qualität von erfassten Magnetresonanzdaten, in Abhängigkeit verschiedener Anforderungen verbessert oder optimiert werden.

In einer Ausführungsform der erfindungsgemäßen Magnetresonanzvorrichtung ist die Steuereinheit dazu ausgebildet, symmetrische Stromflüsse durch eine Mehrzahl von Gradientenspulen einer ersten Gradienten-Anordnung mittels einer Mehrzahl von Gradienten-Verstärkern der ersten Gradienten-Anordnung in unsymmetrische Stromflüsse zu überführen, um einen Wechsel von einem (reinen) homogenen Magnetfeld zu einem magnetischen Gradientenfeld bereitzustellen.

Insbesondere können die Stromflüsse durch die Mehrzahl der Gradientenspulen jeweils Anteile oder Gradiententerme aufweisen, welche bei Bereitstellen eines homogenen Magnetfelds im Wesentlichen identisch sind, aber sich bei Bereitstellen eines magnetischen Gradientenfelds unterscheiden. Ein Gradiententerm kann dabei durch einen Anteil des Stromflusses durch eine Gradientenspule charakterisiert sein oder einen Anteil eines Stromflusses durch eine Gradientenspule konstituieren. Bei Vorliegen eines magnetischen Gradientenfelds kann ein erster Gradiententerm einer ersten Gradientenspule beispielsweise ein negatives Vorzeichen aufweisen, während ein zweiter Gradiententerm einer zweiten Gradientenspule ein positives Vorzeichen aufweist. Eine Differenz zwischen den Stromflüssen durch die erste Gradientenspule und die zweite Gradientenspule kann in diesem Fall durch eine Summe der Beträge der Gradiententerme der erste Gradientenspulen und der zweiten Gradientenspule charakterisiert sein. Hingegen kann eine Differenz zwischen den Stromflüssen durch die erste Gradientenspule und die zweite Gradientenspule bei Bereitstellen eines homogenen Magnetfelds im Wesentlichen dem Wert Null entsprechen.

Durch Bereitstellen einer Steuereinheit, welche dazu ausgebildet ist, geringe Anteile von Stromflüsse durch eine Mehrzahl von Gradientenspulen zu verändern oder zu invertieren, lässt sich ein Schalten hoher Ströme bei einem Übergang eines homogenen Magnetfelds zu einem magnetischen Gradientenfeld auf vorteilhafte Weise vermeiden.

Gemäß einer weiteren Ausführungsform ist die Steuereinheit der erfindungsgemäßen Magnetresonanzvorrichtung dazu ausgebildet, wenigstens einen Gradienten-Verstärker einer ersten Gradienten-Anordnung anzusteuern einen Stromfluss durch die erste Gradienten-Anordnung einzustellen, um ein magnetisches Gradientenfeld mit einer ersten Ausrichtung bereitzustellen und zeitgleich eine Mehrzahl weiterer Gradienten-Verstärker zumindest einer weiteren Gradienten-Anordnung anzusteuern, ein im Wesentlichen homogenes Magnetfeld bereitzustellen, wobei sich das im Wesentlichen homogene Magnetfeld in der Bildgebungsregion der Magnetanordnung mit dem magnetischen Gradientenfeld überlagert.

Es ist vorstellbar, dass der wenigstens eine Gradienten-Verstärker genau eine Gradientenspule der ersten Gradienten-Anordnung mit einem Stromfluss beaufschlagt, um das magnetische Gradientenfeld hinsichtlich der ersten Raumrichtung bereitzustellen. Vorzugsweise ist die Steuereinheit dazu ausgebildet, einen ersten Gradienten-Verstärker und einen zweiten Gradienten-Verstärker der ersten Gradienten-Anordnung anzusteuern, eine erste Gradientenspule und eine zweite Gradientenspule der ersten Gradienten-Anordnung mit unsymmetrischen Stromflüssen zu beaufschlagen, um das magnetische Gradientenfeld bereitzustellen.

In einer Ausführungsform ist die Steuereinheit dazu ausgebildet, zeitgleich eine Mehrzahl weiterer Gradienten-Verstärker zumindest einer weiteren Gradienten-Anordnung anzusteuern, ein im Wesentlichen homogenes Magnetfeld bereitzustellen. Dies kann bedeuten, dass der Stromfluss durch die zumindest eine weitere Gradienten-Anordnung synchron oder zeitgleich zu dem Stromfluss durch die erste Gradienten-Anordnung bereitgestellt wird. Der Stromfluss durch die zumindest eine weitere Gradienten-Anordnung kann sich insbesondere zeitlich mit dem Stromfluss durch die erste Gradienten-Anordnung überschneiden.

In einer bevorzugten Ausführungsform ist die Steuereinheit dazu ausgebildet, eine Mehrzahl weiterer Gradienten-Verstärker einer zweiten Gradienten-Anordnung und einer dritten Gradienten-Anordnung zeitgleich anzusteuern, um das im Wesentlichen homogene Magnetfeld bereitzustellen.

Durch das Ansteuern der Mehrzahl von Gradienten-Verstärkern der zweiten Gradienten-Anordnung und der dritten Gradienten-Anordnung lässt sich eine magnetische Feldstärke des im Wesentlichen homogenen Magnetfelds auf vorteilhafte Weise erhöhen. Dadurch kann eine Qualität erfasster Magnetresonanzdaten vorteilhaft verbessert werden.

Weitere Vorteile und Einzelheiten ergeben sich aus der nachfolgenden Beschreibung von Ausführungsbeispielen in Verbindung mit den Zeichnungen. Es zeigen in Prinzipdarstellung:
- Fig. 1: eine konventionelle Magnetresonanzvorrichtung,
- Fig. 2: eine schematische Darstellung von Stromflüssen in einem konventionellen Gradientensystem,
- Fig. 3: eine mögliche Ausführungsform einer erfindungsgemäßen Magnetanordnung,
- Fig. 4: eine mögliche Ausführungsform einer erfindungsgemäßen Magnetanordnung,
- Fig. 5: eine mögliche Ausführungsform einer erfindungsgemäßen Magnetanordnung,
- Fig. 6: eine schematische Darstellung von Stromflüssen in Gradienten-Anordnungen einer erfindungsgemäßen Magnetanordnung,
- Fig. 7: eine mögliche Ausführungsform einer erfindungsgemäßen Magnetanordnung.

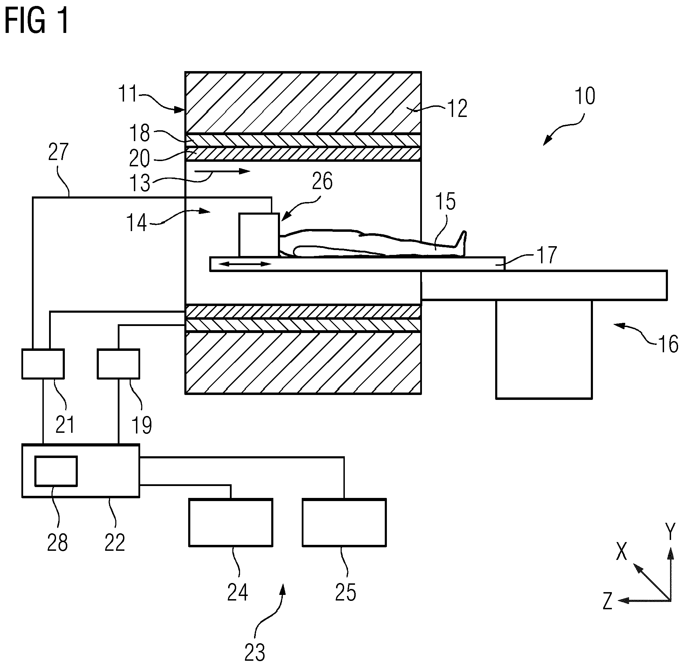

In Fig. 1 ist eine mögliche Ausführungsform einer konventionellen Magnetresonanzvorrichtung 10 schematisch dargestellt. Die Magnetresonanzvorrichtung 10 umfasst eine Magneteinheit 11, welche z. B. einen Permanentmagneten, einen Elektromagneten oder einen supraleitenden Hauptmagneten 12 zur Erzeugung eines statischen und homogenen Grundmagnetfelds 13 (B0-Magnetfeld) aufweist. Zudem umfasst die Magnetresonanzvorrichtung 10 einen Patientenaufnahmebereich 14 zu einer Aufnahme eines Patienten 15. Der Patientenaufnahmebereich 14 ist in dem vorliegenden Ausführungsbeispiel zylinderförmig ausgestaltet und in einer Umfangsrichtung von der Magneteinheit 11 umgeben. Der Patientenaufnahmebereich 14 stimmt im Wesentlichen mit einer Bildgebungsregion der Magnetresonanzvorrichtung 10 überein.

Der Patient 15 kann mittels einer Patientenlagerungsvorrichtung 16 der Magnetresonanzvorrichtung 10 in dem Patientenaufnahmebereich 14 positioniert werden. Die Patientenlagerungsvorrichtung 16 weist hierfür einen innerhalb des Patientenaufnahmebereichs 14 bewegbar ausgestalteten Patiententisch 17 auf. Die Magneteinheit 11 weist weiterhin eine Gradientenspule 18 zum Erzeugen von magnetischen Gradientenfeldern auf, welche für eine Ortskodierung während einer Magnetresonanzmessung verwendet wird. Die Gradientenspule 18 wird mittels einer Gradientensteuereinheit 19 der Magnetresonanzvorrichtung 10 angesteuert. Die Gradientensteuereinheit 19 kann einen Gradienten-Verstärker 29 (nicht gezeigt) aufweisen, welcher dazu ausgebildet ist, einen Stromfluss in der Gradientenspule 18 bereitzustellen. Die Magneteinheit 11 kann weiterhin eine Hochfrequenzantenne umfassen, welche im vorliegenden Ausführungsbeispiel als fest in die Magnetresonanzvorrichtung 10 integrierte Körperspule 20 ausgebildet ist. Die Körperspule 20 ist zu einer Anregung von Atomkernen ausgelegt, die sich in dem von dem Hauptmagneten 12 erzeugten Grundmagnetfeld 13 befinden. Die Körperspule 20 wird von einer Hochfrequenzeinheit 21 der Magnetresonanzvorrichtung 10 angesteuert und strahlt hochfrequente Signale in einen Untersuchungsraum ein, der im Wesentlichen von einem Patientenaufnahmebereich 14 der Magnetresonanzvorrichtung 10 gebildet ist. Die Körperspule 20 kann weiterhin auch zu einem Empfangen von Magnetresonanzsignalen ausgebildet sein.

Für eine Steuerung des Hauptmagneten 12, der Gradientensteuereinheit 19 und der Hochfrequenzeinheit 21 weist die Magnetresonanzvorrichtung 10 eine Steuereinheit 22 auf. Die Steuereinheit 22 ist dazu ausgebildet, eine Durchführung einer Sequenz, wie z. B. einer bildgebenden Gradientenechosequenz, einer TSE-Sequenz oder einer UTE-Sequenz, zu steuern. Zudem umfasst die Steuereinheit 22 eine Auswerteeinheit 28 zu einer Auswertung von digitalisierten Magnetresonanzsignalen, die während einer Magnetresonanzmessung erfasst werden.

Des Weiteren umfasst die Magnetresonanzvorrichtung 10 eine Benutzerschnittstelle 23, welche eine Signalverbindung mit der Steuereinheit 22 aufweist. Steuerinformationen, wie beispielsweise Bildgebungsparameter und rekonstruierte Magnetresonanzbilder, können auf einer Anzeigeeinheit 24, beispielsweise auf zumindest einem Monitor der Benutzerschnittstelle 23, für einen Nutzer angezeigt werden. Weiterhin weist die Benutzerschnittstelle 23 eine Eingabeeinheit 25 auf, mittels der Parameter einer Magnetresonanzbildgebung von dem Nutzer eingegeben werden können.

Ferner weist die Magnetresonanzvorrichtung 10 eine Lokalspule 26 auf, welche vorliegend an einem Kopf des Patienten 15 positioniert ist und Magnetresonanzsignale aus einem Volumen einer Kopfregion an die Magnetresonanzvorrichtung 10 überträgt. Die Lokalspule 26 weist vorzugsweise eine elektrische Anschlussleitung 27 auf, welche eine Signalverbindung mit der Hochfrequenzeinheit 21 und der Steuereinheit 22 bereitstellt. Die Lokalspule 26 kann aber auch mittels einer drahtlosen Signalverbindung mit der Magnetresonanzvorrichtung 10 verbunden sein. Ebenso wie die Körperspule 20 kann auch die Lokalspule 26 zu einer Anregung von Atomkernen und zu einem Empfangen von Magnetresonanzsignalen ausgebildet sein. Zum Aussenden von hochfrequenten Signalen wird eine Sendeeinheit der Lokalspule 26 von der Hochfrequenzeinheit 21 angesteuert. Die Lokalspule 26 kann den Kopf des Patienten 15 außenumfänglich entlang einer Längsachse des Patienten 15 umschließen. Die Sendeeinheit und/oder eine Empfangseinheit der Lokalspule 26 können insbesondere von einem Halteelement 33 getragen werden, welches relativ zu einem Basiselement der Lokalspule 26 positionierbar ist.

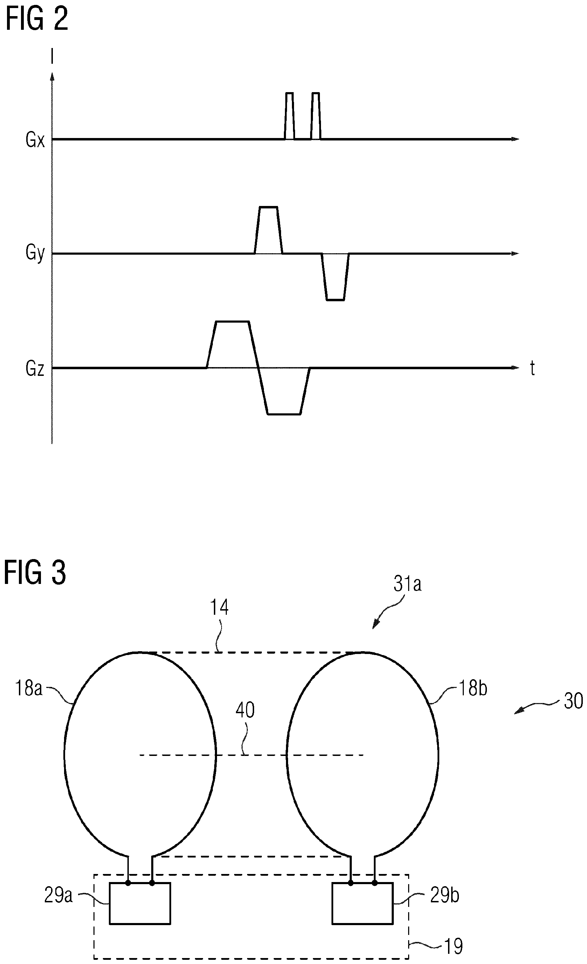

Konventionelle Magnetresonanzvorrichtungen 10 weisen typischerweise drei Gradientenspulen 18 auf, denen jeweils genau ein Gradienten-Verstärker zugeordnet ist. Zur Erzeugung von magnetischen Gradientenfeldern in X-, Y-, und Z-Richtung während einer Magnetresonanzuntersuchung werden die Gradientenspulen 18 mittels der Gradientensteuereinheit zum Bereitstellen der magnetischen Gradientenfelder Gx, Gy und Gz angeregt. Fig. 2 zeigt einen typischen Stromfluss durch die Gradientenspulen 18. Die magnetischen Gradientenfelder in X-, Y- und Z-Richtung werden dabei dem statischen und homogenen Magnetfeld des Hauptmagneten 12 überlagert.

Fig. 3 zeigt eine Ausführungsform einer erfindungsgemäßen Magnetanordnung 30 mit zwei Gradientenspulen 18a und 18b (18a-b). Die Gradientenspulen 18a-b sind im Wesentlichen kreisförmig und planar in parallel ausgerichteten, voneinander getrennten Ebenen angeordnet, sodass eine Projektion einer von der ersten Gradientenspule 18a umschlossenen, ersten Fläche entlang eines Normalenvektors der ersten Fläche und eine von der zweiten Gradientenspule 18b umschlossenen, zweiten Fläche, eine nichtleere Schnittmenge aufweisen.

Die Gradientenspulen 18a-b sind bevorzugt an gegenüberliegenden Enden eines im Wesentlichen zylinderförmig ausgestalteten Patientenaufnahmebereichs 14 angeordnet. Die Gradientenspulen 18a-b können den zylinderförmigen Patientenaufnahmebereich 14 zumindest abschnittweise entlang einer Umfangsrichtung umschließen.

Die Gradientenspule 18a ist elektrisch mit dem Gradienten-Verstärker 29a verbunden. Der Gradienten-Verstärker 29a ist dazu ausgebildet, einen Stromfluss in der Gradientenspule 18a bereitzustellen. Es ist vorstellbar, dass der Gradienten-Verstärker 29a hierfür eine Signalverbindung mit der Steuereinheit 22 aufweist (nicht gezeigt), welche dazu ausgebildet ist, den Gradienten-Verstärker 29a mittels der Signalverbindung entsprechend anzusteuern. Gleichermaßen ist die Gradientenspule 18b elektrisch mit dem Gradienten-Verstärker 29b verbunden. Auch der Gradienten-Verstärker 29b kann eine Signalverbindung mit der Steuereinheit 22 aufweisen (nicht gezeigt). Der Gradienten-Verstärker 29a und der Gradienten-Verstärker 29b, aber auch weitere Gradienten-Verstärker 29, können Teil einer Gradientensteuereinheit 19 sein oder separat voneinander vorliegen.

Die Gradientenspulen 18a-b können insbesondere als ein Helmholtz-Paar ausgestaltet sein. Für die Erzeugung eines homogenen Magnetfelds können die Gradientenspulen 18a-b symmetrisch oder gleichsinnig mittels der Gradienten-Verstärker 29a und 29b bestromt sein. Zum Bereitstellen eines magnetischen Gradientenfelds entlang der Zylinderachse des Patientenaufnahmebereichs 14 (z. B. Z-Richtung) kann sich der Stromfluss durch die Gradientenspule 18a von dem Stromfluss durch die Gradientenspule 18b unterscheiden. Insbesondere können die Stromflüsse durch die beiden Gradientenspulen 18a-b jeweils Anteile oder Gradiententerme aufweisen, welche sich bei Bereitstellung eines magnetischen Gradientenfelds unterscheiden. Beispielsweise kann der Gradiententerm der Gradientenspule 18a ein negatives Vorzeichen aufweisen, während der Gradiententerm der Gradientenspule 18b ein positives Vorzeichen aufweist. Eine Differenz zwischen den Stromflüssen durch die Gradientenspule 18a und die Gradientenspule 18b kann in diesem Fall durch die Summe der Beträge der Gradiententerme der Gradientenspulen 18a-b charakterisiert sein. Hingegen kann die Differenz zwischen den Stromflüssen durch die Gradientenspulen 18a-b bei Bereitstellung eines homogenen Magnetfelds im Wesentlichen dem Wert Null entsprechen.

Es ist weiterhin vorstellbar, dass die in Fig. 3 gezeigte Gradienten-Anordnung 31a eine dritte Gradientenspule 18c (nicht gezeigt) aufweist, welche mittig zwischen den Gradientenspulen 18a-b entlang der Zylinderachse 40 angeordnet ist. Die Gradienten-Anordnung 31a kann in diesem Fall insbesondere als eine Maxwell-Spulenanordnung ausgestaltet sein.

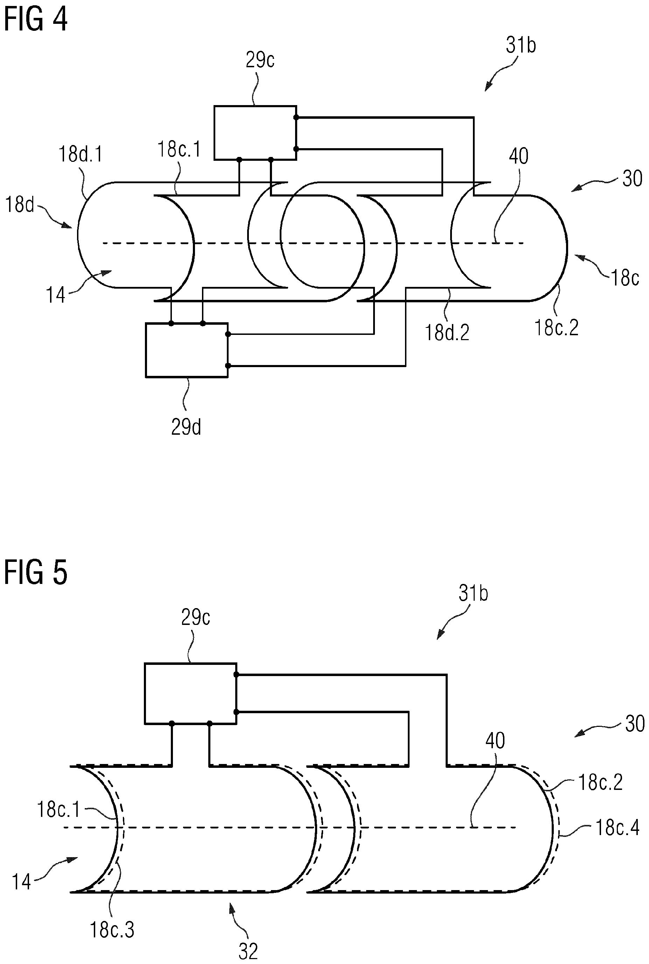

In Fig. 4 ist einer weitere Ausführungsform der erfindungsgemäßen Magnetanordnung 30 dargestellt. Die Magnetanordnung 30 weist in dem gezeigten Beispiel eine Gradienten-Anordnung 31b mit den Gradientenspulen 18c und 18d (18c-d) auf, welche vorliegend als Sattelspulen ausgestaltet sind. Die Gradientenspulen 18c und 18d sind elektrisch unverbunden und entlang einer axialen Richtung der hohlzylinderförmigen Magnetanordnung 30 einander gegenüberliegend angeordnet. Die hohlzylinderförmige Magnetanordnung 30 umschließt hierbei den zylinderförmigen Patientenaufnahmebereich 14 entlang der Zylinderachse 40 zumindest teilweise oder vollständig. Beispielsweise können die Gradientenspulen 18a-b die hohlzylinderförmige Magnetanordnung 30 in eine linke Hälfte und eine Rechte Hälfte, bzw. eine untere Hälfte und eine obere Hälfte zu unterteilen. Die linke Hälfte und die rechte Hälfte bzw. die untere Hälfte und die obere Hälfte können dabei symmetrisch ausgestaltet sein oder im Wesentlichen übereinstimmende Abmessungen aufweisen.

Die Gradientenspulen 18c und 18d sind vorliegend in jeweils zwei Spulenabschnitte 18c.1 und 18c.2 sowie 18d.1 und 18d.2 unterteilt. Die Spulenabschnitte 18c.1 und 18c.2 der Gradientenspule 18c sind entlang der Zylinderachse 40 aufeinanderfolgend oder hintereinander in der zylinderförmigen Magnetanordnung 30 angeordnet. Gleichermaßen können auch die Spulenabschnitte 18d.1 und 18d.2 der Gradientenspule 18d entlang der Zylinderachse 40 hintereinander in der zylinderförmigen Magnetanordnung 30 angeordnet sein. Die Spulenabschnitte 18c.1 und 18c.2 sind bevorzugt entlang einer Schnittebene durch die Zylinderachse 40 spiegelsymmetrisch zu den Spulenabschnitten 18d.1 und 18d.2 angeordnet. Die Spulenabschnitte 18c.1 und 18c.2 sind elektrisch mit dem Gradienten-Verstärker 29c verbunden, während die Spulenabschnitte 18d.1 und 18d.2 elektrisch mit dem Gradienten-Verstärker 29d verbunden sind.

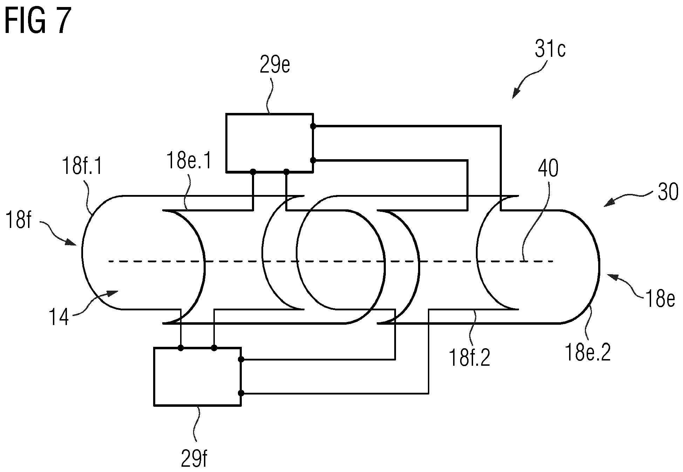

Wie in Fig. 7 dargestellt, kann die erfindungsgemäße Magnetanordnung 30 eine weitere Gradienten-Anordnung 31c umfassen. Die Gradienten-Anordnung 31c kann Gradientenspulen 18e und 18f aufweisen, welche wie in Fig. 4 gezeigt, in Spulenabschnitte 18e.1 und 18e.2 sowie 18f.1 und 18f.2 unterteilt sein können. Vorzugsweise sind auch die Gradientenspulen 18e und 18f als Sattelspulen ausgestaltet und in axialer Richtung der hohlzylinderförmigen Magnetanordnung 30 einander gegenüberliegend angeordnet. Analog zu der Gradienten-Anordnung 31b können auch die Gradientenspulen 18e und 18f elektrisch mit dedizierten Gradienten-Verstärkern 29e und 29f verbunden sein. Die Gradienten-Anordnung 31c ist bevorzugt um etwa 90° gegenüber der Gradienten-Anordnung 31b rotiert in der Magnetanordnung 30 angeordnet.

Alternativ zu der in Fig. 4 dargestellten Ausführungsform können die Gradientenspulen auch als Segmentspulen ausgestaltet sein.

Fig. 5 zeigt eine Ausführungsform der erfindungsgemäßen Magnetanordnung 30, bei welcher die Gradientenspule 18c der Gradienten-Anordnung 31b eine Sekundärspule 32 mit den Spulenabschnitten 18c.3, 18c.4 aufweist. Die Spulenabschnitte 18c.3, 18c.4 der Sekundärspule 32 sind im Wesentlichen parallel zu den Spulenabschnitten 18c.1 und 18c.2 der Gradientenspule 18c angeordnet und begrenzen diese entlang einer von dem Patientenaufnahmebereich 14 abgewandten Richtung. Die Sekundärspule 32 ist dazu ausgebildet, ein magnetisches Feld in dem Patientenaufnahmebereich 14 bereitzustellen. Die Sekundärspule 32 unterscheidet sich somit von einer elektromagnetischen Schirmung bzw. einer Schirmungslage, wie sie bei konventionellen Magnetresonanzvorrichtungen (siehe Fig. 1) eingesetzt wird.

In einer bevorzugten Ausführungsform sind die Spulenabschnitte 18c.3 und 18c.4 der Sekundärspule 32 und die Spulenabschnitte 18c.1 und 18c.2 der Gradientenspule 18c gemeinsam elektrisch mit dem Gradienten-Verstärker 29c verbunden. Es ist aber ebenso vorstellbar, dass die Spulenabschnitte 18c.3 und 18c.4 der Sekundärspule 32 und die Spulenabschnitte 18c.1 und 18c.2 der Gradientenspule 18c elektrisch mit separaten Gradienten-Verstärker verbunden sind. Weiterhin kann jeder Spulenabschnitt 18c.1 bis 18c.4 elektrisch mit einem separaten oder dedizierten Gradienten-Verstärker 29 verbunden sein.

In einer alternativen Ausgestaltungsform liegt die Gradientenspule 18c einstückig bzw. ungeteilt, d. h. nicht in einzelnen Spulenabschnitten 18c.1 und 18c.2, vor. Gleichermaßen kann auch die Sekundärspule einstückig vorliegen. Es ist jedoch ebenso vorstellbar, dass die Gradientenspule 18c und die Sekundärspule 32 jeweils in mehr als zwei Spulenabschnitte untergliedert sind.

Es ist vorstellbar, dass jede Gradientenspule 18 gemäß einer oben beschriebenen Ausführungsform eine Sekundärspule 32 aufweist. Insbesondere können die Gradientenspule 18d, aber auch die Gradientenspulen 18e und/oder 18f der Gradienten-Anordnung 31c (nicht gezeigt), Sekundärspulen 32 aufweisen. Es ist ferner vorstellbar, dass auch die Gradientenspulen 18a und 18b (siehe Fig. 3) Sekundärspulen 32 aufweisen, welche jeweils im Wesentlichen parallel zu den Gradientenspulen 18a und 18b angeordnet sind und diese in einer von dem Patientenaufnahmebereich 14 abgewandten Richtung begrenzen.

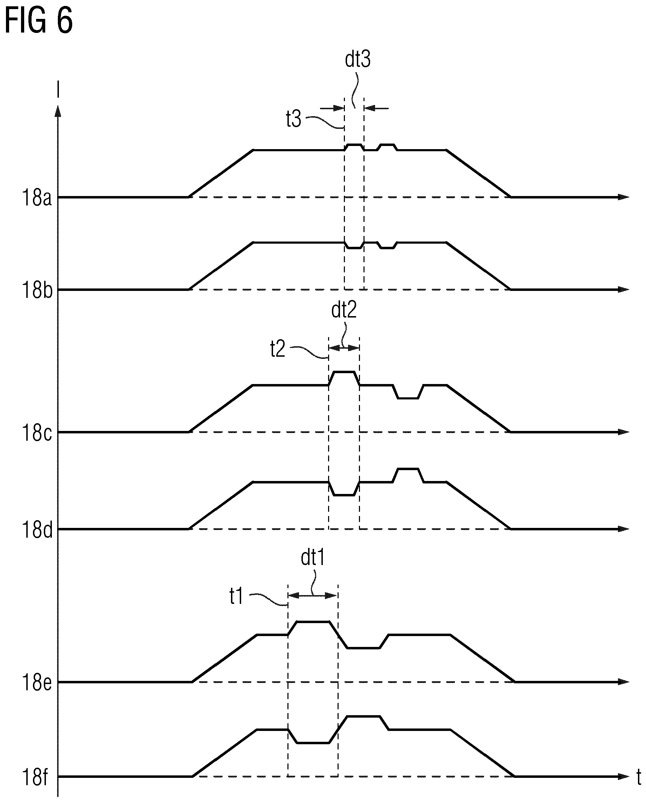

Fig. 6 zeigt schematisch, wie die Gradienten-Anordnungen 31a, 31b und 31c (31a-c) der erfindungsgemäßen Magnetanordnung 30 mittels einer Mehrzahl von Gradienten-Verstärkern ansteuerbar sind, um abwechselnd ein homogenes Magnetfeld und ein magnetisches Gradientenfeld oder ein homogenes Magnetfeld mit überlagertem magnetischen Gradientenfeld bereitzustellen.

In einer bevorzugten Ausführungsform weisen die Gradienten-Anordnungen 31a-c jeweils zwei Gradientenspulen 18 und zwei Gradienten-Verstärker 29 auf. Dabei ist jeder Gradienten-Verstärker 29a-f (nicht gezeigt) elektrisch mit einer Gradientenspule 18a-f gemäß einer oben beschriebenen Ausführungsform verbunden.

Vorzugsweise wird ein homogenes Magnetfeld bereitgestellt, indem sich die Magnetfelder der Gradienten-Anordnungen 31a-c überlagern. Es ist vorstellbar, dass jede Gradienten-Anordnung 31a, 31b und 31c ein im Wesentlichen homogenes Magnetfeld bereitstellt, welche sich in dem Patientenaufnahmebereich 14 überlagern. Eine magnetische Feldstärke des homogenen Magnetfelds kann sich dabei durch Addition aus den magnetischen Feldstärken der Magnetfelder der Gradienten-Anordnungen 31a-c ergeben. Für das Bereitstellen der homogenen Magnetfelder können jeweils zwei Gradientenspulen 18a und 18b, 18c und 18d sowie 18e und 18f der Gradienten-Anordnungen 31a-c mittels der Gradienten-Verstärker 29a und 29b, 29c und 29 sowie 29e und 29f symmetrisch oder gleichsinnig mit einem Stromfluss I beaufschlagt werden. Beispielsweise können die Gradientenspulen 18a und 18b mittels der Gradienten-Verstärker 29a und 29b mit einem gleichsinnigen Stromfluss I beaufschlagt werden. Gleichermaßen können die Gradientenspulen 18c und 18d mittels der Gradienten-Verstärker 29c und 29d mit einem symmetrischen Stromfluss I versorgt werden.

In dem in Fig. 6 gezeigten Beispiel erzeugen die Gradientenspulen 18a und 18b ein homogenes Magnetfeld in dem Patientenaufnahmebereich 14, wenn die Gradienten-Verstärker 29a und 29b einen symmetrischen Stromfluss I in den Gradientenspulen 18a und 18b bereitstellen. Die Gradientenspulen 18a und 18b können hierbei insbesondere als Helmholtz-Spulen agieren. Der Stromfluss I durch die Gradientenspulen 18a und 18b kann Flanken aufweisen, welche aus einem Hochfahr- und Herunterfahrverhalten der Gradienten-Verstärker 29a und 29b resultieren können. Der zeitabhängige Stromfluss I kann daher annähernd eine Trapezform aufweisen.

Zur Erzeugung des homogenen Magnetfelds können zeitgleich jeweils symmetrische Stromflüsse I in den Gradientenspulen 18c und 18d sowie 18e und 18f bereitgestellt werden. Die von den Gradientenspulen 18a-f erzeugten, homogenen Magnetfelder überlagern sich dabei im Patientenaufnahmebereich 14.

Es ist ebenso vorstellbar, dass die von den Gradienten-Anordnungen 31a, 31b und 31c bereitgestellten Magnetfelder Gradienten aufweisen (magnetische Gradientenfelder), welche sich insgesamt zu einem homogenen Magnetfeld in dem Patientenaufnahmebereich 14 überlagern.

Es ist weiterhin vorstellbar, dass nur eine Gradienten-Anordnung 31 oder zwei Gradienten-Anordnungen 31 ein homogenes Magnetfeld in dem Patientenaufnahmebereich 14 bereitstellen. In diesem Fall können die Gradientenspulen 18 der einen Gradienten-Anordnung 31 oder der beiden Gradienten-Anordnungen 31, welche kein homogenes Magnetfeld bereitstellen, unsymmetrisch mit einem Stromfluss I beaufschlagt sein oder keinen Stromfluss I aufweisen. Beispielsweise werden die Gradientenspulen 18a-b und 18c-d mittels der Gradienten-Verstärker 29a-b und 29c-d jeweils mit symmetrischen Stromflüssen I beaufschlagt, um ein homogenes Magnetfeld in dem Patientenaufnahmebereich 14 bereitzustellen. Die Gradientenspulen 18e-f sind dagegen mittels der Gradienten-Verstärker 29e-f mit unsymmetrischen Stromflüssen I beaufschlagt, um ein magnetisches Gradientenfeld mit einer Orientierung in der X-Richtung in dem Patientenaufnahmebereich 14 bereitzustellen.

In Fig. 6 ist eine bevorzugte Ausführungsform der erfindungsgemäßen Magnetanordnung 30 dargestellt, bei welcher die Gradienten-Anordnungen 31a-c an ausgewählten Zeitpunkten gemeinsam mit einem Stromfluss I beaufschlagt sind, um ein homogenes Magnetfeld in dem Patientenaufnahmebereich 14 bereitzustellen. Für die räumliche Kodierung von erfassten Magnetresonanzsignalen während einer Bildgebungssequenz werden jedoch üblicherweise kurzlebige magnetische Gradientenfelder entlang der drei Raumrichtungen eines Kartesischen Koordinatensystems benötigt. Daher sind die Gradienten-Verstärker 29a-f der Gradienten-Anordnungen 31a-c vorliegend dazu ausgebildet, einen geringen Anteil des Stromflusses I (Gradiententerm) in gegenüberliegenden Gradientenspulen 18 einer Gradienten-Anordnung 31 kurzfristig zu invertieren, um ein magnetisches Gradientenfeld in einer gewünschten Raumrichtung zu erzeugen.

Wie in Fig. 6 beispielhaft gezeigt, kann der Stromfluss I durch die Gradientenspule 18c mittels des Gradienten-Verstärkers 29c zu einem Zeitpunkt t2 kurzfristig angehoben werden, während der Stromfluss I durch die Gradientenspule 18d mittels des Gradienten-Verstärkers 29d zu dem Zeitpunkt t2 kurzfristig abgesenkt wird. Dadurch kann ein magnetisches Gradientenfeld mit einer Ausrichtung in X-Richtung bereitgestellt werden, ohne eine signifikante Veränderung der magnetischen Feldstärke des zugrundeliegenden, homogenen Magnetfelds zu verursachen. Gleichermaßen kann ein Stromfluss durch die Gradientenspule 18e mittels des Gradienten-Verstärkers 29e zu einem Zeitpunkt t1 kurzfristig angehoben werden, während der Stromfluss I durch die Gradientenspule 18f mittels des Gradienten-Verstärkers 29f zu dem Zeitpunkt t1 kurzfristig abgesenkt wird, um ein magnetisches Gradientenfeld mit einer Ausrichtung in Y-Richtung bereitzustellen.

Auf eine ähnliche Weise lässt sich ein magnetisches Gradientenfeld Z-Richtung mittels der erfindungsgemäßen Magnetanordnung 30 bereitstellen. Ein Zeitpunkt ti, zu welchem ein Anteil des Stromflusses I durch gegenüberliegende Gradientenspulen 18 einer Gradienten-Anordnung 31 kurzfristig invertiert wird, kann dabei in Abhängigkeit einer durchzuführenden Magnetresonanzuntersuchung oder Bildgebungssequenz vorgegeben sein. Die Zeitpunkte t1, t2, t3 können sich dabei unterscheiden. Es ist aber ebenso vorstellbar, dass zwei oder drei der Zeitpunkte t1, t2 und t3 zusammenfallen.

Die Zeitspannen dt1, dt2 und dt3 können Zeiträume charakterisieren, in denen die Stromflüsse I mittels der Gradienten-Verstärker variiert werden, um ein magnetisches Gradientenfeld bereitzustellen. Es ist vorstellbar, dass sich die Zeitspannen dt1, dt2 und dt3 zumindest teilweise zeitlich überlagern. Die Zeitspannen dt1, dt2 und dt3 können aber auch zeitlich aufeinanderfolgen oder zeitlich voneinander beabstandet sein. In einer Ausführungsform unterscheiden sich die Dauern der Zeiträume dt1, dt2 und dt3.

Selbstverständlich sind die in Fig. 6 dargestellten Stromflüsse I durch die Gradientenspulen 18a-f als rein exemplarisch zu verstehen. Die Stromflüsse I sowie die erzeugten Magnetfelder können in Abhängigkeit der Bildgebungssequenz, der Magnetresonanzuntersuchung, aber auch eines untersuchten Objekts und/oder einer spezifischen Geometrie der Magnetresonanzvorrichtung, variieren.

Es ist insbesondere vorstellbar, dass die Geometrie der Magnetresonanzvorrichtung von einer konventionellen Zylinderform abweicht. Beispielsweise die Magnetresonanzvorrichtung als ein "C"-Typ oder ein "offener" Scanner ausgeführt sein. Die Gradientenspulen können dementsprechend eine im Wesentlichen planare Gestalt aufweisen. Bevorzugt sind die Gradientenspulen in diesem Fall auf zwei im Wesentlichen gegenüberliegenden Seiten des Bildgebungsvolumens oder des Patientenaufnahmebereichs 14 angeordnet und weisen angepasste Wicklungsmuster auf, um magnetische Gradientenfelder in gewünschten Raumrichtungen zu erzeugen. Beispielsweise sind eine erste Gradientenspule mit einem ersten Gradienten-Verstärker einer ersten Gradienten-Anordnung und eine zweite Gradientenspule mit einem zweiten Gradienten-Verstärker der ersten Gradienten-Anordnung auf gegenüberliegenden Seiten eines Bildgebungsvolumens bzw. des Patientenaufnahmebereichs 14 der Magnetanordnung 30 angeordnet und räumlich voneinander beabstandet.

Die erfindungsgemäße Magnetanordnung 30 kann selbstverständlich weitere Komponenten umfassen, welche Magnetresonanzvorrichtungen 10 üblicherweise aufweisen. Es ist ebenso vorstellbar, dass die Magnetresonanzvorrichtung 10 statt des zylinderförmigen Aufbaus einen C-förmigen, einen dreieckigen oder einen asymmetrischen Aufbau der Magnetfeld-erzeugenden Komponenten aufweist. Die Magnetresonanzvorrichtung 10 kann insbesondere eine dedizierte Magnetresonanzvorrichtung 10 sein, welche dazu ausgebildet ist, eine Magnetresonanzbildgebung der Kieferregion eines stehenden oder sitzenden Patienten 15 durchzuführen.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen.

## Patentansprüche

1. Magnetanordnung (30) für eine Magnetresonanzvorrichtung (10) zum Erfassen von Magnetresonanzdaten eines Objekts, umfassend zumindest eine Gradienten-Anordnung (31) mit einer Mehrzahl von Gradientenspulen und einer Mehrzahl von Gradienten-Verstärkern, wobei die Mehrzahl von Gradienten-Verstärkern dazu ausgebildet ist, einen Stromfluss in der Mehrzahl von Gradientenspulen variabel einzustellen, wobei jede Gradientenspule (18) der zumindest einen Gradienten-Anordnung (31) jeweils elektrisch mit einem Gradienten-Verstärker (29) der zumindest einen Gradienten-Anordnung (31) verbunden ist und wobei die Magnetanordnung (30) dazu ausgebildet ist, mittels der Mehrzahl von Gradienten-Verstärkern abwechselnd ein im Wesentlichen homogenes Magnetfeld sowie ein magnetisches Gradientenfeld bereitzustellen.

2. Magnetanordnung (30) nach Anspruch 1, wobei die zumindest eine Gradienten-Anordnung (31) eine erste Gradientenspule (18a) und eine zweite Gradientenspule (18b) umfasst, wobei die erste Gradientenspule (18a) und die zweite Gradientenspule (18b) im Wesentlichen kreisförmig und planar in parallel ausgerichteten, voneinander getrennten Ebenen angeordnet sind, sodass eine Projektion einer von der ersten Gradientenspule (18a) umschlossenen, ersten Fläche entlang eines Normalenvektors der ersten Fläche und eine von der zweiten Gradientenspule (18b) umschlossenen, zweiten Fläche, eine nichtleere Schnittmenge aufweisen, wobei die erste Gradientenspule (18a) elektrisch mit einem ersten Gradienten-Verstärker (29a) verbunden ist und wobei die zweite Gradientenspule (18b) elektrisch mit einem zweiten Gradienten-Verstärker (29b) verbunden ist.

3. Magnetanordnung (30) nach einem der Ansprüche 1 oder 2, ferner umfassend eine zweite Gradienten-Anordnung (31b) mit einer Mehrzahl von zweiten Gradientenspulen und einer Mehrzahl von zweiten Gradienten-Verstärkern, wobei wenigstens zwei Gradientenspulen (18c, 18d) der Mehrzahl von zweiten Gradientenspulen jeweils elektrisch mit einem Gradienten-Verstärker (29c, 29d) der Mehrzahl von zweiten Gradienten-Verstärkern verbunden sind und wobei die Magnetanordnung (30) dazu ausgebildet ist, mittels der Mehrzahl von zweiten Gradienten-Verstärkern abwechselnd ein im Wesentlichen homogenes Magnetfeld sowie ein zweites magnetisches Gradientenfeld bereitzustellen, wobei sich eine Ausrichtung des zweiten magnetischen Gradientenfelds von einer Ausrichtung des magnetischen Gradientenfelds unterscheidet.

4. Magnetanordnung (30) nach Anspruch 3, ferner umfassend eine dritte Gradienten-Anordnung (31c), mit einer Mehrzahl von dritten Gradientenspulen und einer Mehrzahl von dritten Gradienten-Verstärkern, wobei wenigstens zwei Gradientenspulen (18e, 18f) der Mehrzahl von dritten Gradientenspulen jeweils elektrisch mit einem Gradienten-Verstärker (29e, 29f) der Mehrzahl von dritten Gradienten-Verstärkern verbunden sind und wobei die Magnetanordnung (30) dazu ausgebildet ist, mittels der Mehrzahl von dritten Gradienten-Verstärkern abwechselnd ein im Wesentlichen homogenes Magnetfeld sowie ein drittes magnetisches Gradientenfeld bereitzustellen, wobei sich eine Ausrichtung des dritten magnetischen Gradientenfelds von einer Ausrichtung des magnetischen Gradientenfelds und/oder des zweiten magnetischen Gradientenfelds unterscheidet.

5. Magnetanordnung (30) nach einem der Ansprüche 3 oder 4, wobei die Mehrzahl von zweiten Gradientenspulen und/oder die Mehrzahl von dritten Gradientenspulen als Sattelspulen ausgestaltet ist.

6. Magnetanordnung (30) nach einem der Ansprüche 3 oder 4, wobei die Mehrzahl von zweiten Gradientenspulen und/oder die Mehrzahl von dritten Gradientenspulen als Segmentspulen ausgestaltet ist.

7. Magnetanordnung (30) nach einem der Ansprüche 3 bis 6, wobei die Magnetanordnung (30) eine im Wesentlichen hohlzylinderförmige Gestalt aufweist, wobei zumindest eine Gradientenspule (18c) der zweiten Gradienten-Anordnung (31b) einen ersten Spulenabschnitt (18c.1) und einen zweiten Spulenabschnitt (18c.2) umfasst, wobei der erste Spulenabschnitt (18c.1) und der zweite Spulenabschnitt (18c.2) disjunkt voneinander entlang einer axialen Richtung der hohlzylinderförmigen Magnetanordnung (30) aufeinanderfolgend angeordnet sind, wobei der erste Spulenabschnitt (18c.1) und der zweite Spulenabschnitt (18c.2) gemeinsam elektrisch mit genau einem Gradienten-Verstärker (29c) der zweiten Gradienten-Anordnung (31b) verbunden sind.

8. Magnetanordnung (30) nach einem der vorhergehenden Ansprüche, wobei zumindest eine Gradientenspule (18a, 18b, 18c, 18d, 18e, 18f) der zumindest einen Gradienten-Anordnung (31a), der zweiten Gradienten-Anordnung (31b) und/oder der dritten Gradienten-Anordnung (31c) eine Sekundärspule (32) aufweist, wobei die Sekundärspule (32) im Wesentlichen parallel zu der zumindest einen Gradientenspule (18a, 18b, 18c, 18d, 18e, 18f) angeordnet ist und die zumindest eine Gradientenspule (18a, 18b, 18c, 18d, 18e, 18f) in einer von einer Bildgebungsregion der Magnetanordnung (30) abgewandten Richtung begrenzt, wobei die Sekundärspule (32) und die zumindest eine Gradientenspule (18a, 18b, 18c, 18d, 18e, 18f) gemeinsam elektrisch mit einem Gradienten-Verstärker (29a, 29b, 29c, 29d, 29e, 29f) verbunden sind.

9. Magnetanordnung (30) nach einem der vorhergehenden Ansprüche, wobei die zumindest eine Gradienten-Anordnung (31a), die zweite Gradienten-Anordnung (31b) und/oder die dritte Gradienten-Anordnung (31c) elektromagnetisch ungeschirmt sind.

10. Magnetanordnung (30) nach einem der vorhergehenden Ansprüche, wobei die zumindest eine Gradienten-Anordnung (31a), die zweite Gradienten-Anordnung (31b) und/oder die dritte Gradienten-Anordnung (31c) einen Hauptmagneten (12) zur Erzeugung eines statischen, homogenen Magnetfelds ersetzen.

11. Magnetresonanzvorrichtung (10) zum Erfassen von Magnetresonanzdaten eines Objekts, umfassend eine Magnetanordnung (30) nach einem der vorhergehenden Ansprüche und eine Steuereinheit (22), welche dazu ausgebildet ist, eine Mehrzahl von Gradienten-Verstärkern anzusteuern einen Stromfluss in einer Mehrzahl von Gradientenspulen variabel einzustellen, um abwechselnd ein im Wesentlichen homogenes Magnetfeld sowie ein magnetisches Gradientenfeld in einer Bildgebungsregion der Magnetanordnung (30) bereitzustellen.

12. Magnetresonanzvorrichtung (10) nach Anspruch 11, wobei die Steuereinheit (22) dazu ausgebildet ist, symmetrische Stromflüsse durch eine Mehrzahl von Gradientenspulen einer ersten Gradienten-Anordnung (31a, 31b, 31c) mittels einer Mehrzahl von Gradienten-Verstärkern der ersten Gradienten-Anordnung (31a, 31b, 31c) in unsymmetrische Stromflüsse zu überführen, um einen Wechsel von einem homogenen Magnetfeld zu einem magnetischen Gradientenfeld bereitzustellen.

13. Magnetresonanzvorrichtung (10) nach einem der Ansprüche 11 oder 12 mit einer Magnetanordnung (30) nach einem der Ansprüche 3 bis 10, wobei die Steuereinheit (22) dazu ausgebildet ist, wenigstens einen Gradienten-Verstärker (29a, 29b, 29c, 29d, 29e, 29f) einer ersten Gradienten-Anordnung (31a, 31b, 31c) anzusteuern einen Stromfluss durch die erste Gradienten-Anordnung (31a, 31b, 31c) einzustellen, um ein magnetisches Gradientenfeld mit einer ersten Ausrichtung bereitzustellen und zeitgleich eine Mehrzahl weiterer Gradienten-Verstärker zumindest einer weiteren Gradienten-Anordnung anzusteuern, ein im Wesentlichen homogenes Magnetfeld bereitzustellen, wobei sich das im Wesentlichen homogene Magnetfeld in der Bildgebungsregion der Magnetanordnung (30) mit dem magnetischen Gradientenfeld überlagert.

14. Magnetresonanzvorrichtung (10) nach Anspruch 13 mit einer Magnetanordnung (30) nach einem der Ansprüche 4 bis 10, wobei die Steuereinheit (22) dazu ausgebildet ist, eine Mehrzahl weiterer Gradienten-Verstärker einer zweiten Gradienten-Anordnung und einer dritten Gradienten-Anordnung zeitgleich anzusteuern, um das im Wesentlichen homogene Magnetfeld bereitzustellen.
